# EUROPEAN PATENT APPLICATION

(11) **EP 2 055 326 A2**
(43) Date of publication of application: **06.05.2009**
(21) Application number: 08253505.5
(22) Date of filing: 28.10.2008
(51) Int. Cl.: A61L 29/04, A61L 29/02

(54) **Medical device including a metallic substrate component attached to a polymeric component and associated methods**

(30) Priority: 29.10.2007 US 983647 P; 05.09.2008 US 205843
(71) Applicant: Cordis Corporation, Miami Lakes, FL 33014 (US)
(72) Inventor: Velasco, Regina, Fremont California 94536 (US); Bonsignore, Craig, Pleasanton California 94566 (US); MHugo, Scott, Menlo Park California 94025 (US); Maghribi, Mariam, Fremont California 94538 (US)
(74) Representative: Small, Gary James

(57) **Abstract**

A medical device including a medical device metallic substrate component, with perforations therethrough, attached to a medical device polymeric component abutting the thin metallic film component. The medical device also includes a polymeric sleeve covering the perforations and at least a portion of the polymeric component abutting the metallic substrate component. Moreover, the polymeric sleeve intrudes into the perforations and joins the metallic substrate component to the polymeric component. A method for attaching a medical device metallic substrate component to a medical device polymeric component includes forming a plurality of perforations in the medical device metallic substrate component and abutting the medical device polymeric component against the medical device metallic substrate component. Subsequently, a polymeric sleeve is applied over the perforations and at least a portion of the abutted medical device polymeric component. Heat and/or pressure is then applied to the polymer sleeve in a manner that results in the polymer sleeve being joined to the medical device metallic substrate and the portion of the abutted medical device polymeric component and the polymer sleeve flowing into the perforations, thereby attaching the medical device metallic substrate component to the medical device polymeric component.

## Description

### BACKGROUND OF THE INVENTION

Field of the Invention

The present invention relates, in general, to medical devices and, in particular, to medical devices that include a metallic substrate component attached to polymeric component and associated methods.

Description of Related Art

A variety of medical devices (e.g. angioplasty devices, catheters and stents) include both thin metallic film components and polymeric components. For example, angioplasty devices can include a thin metallic film balloon and a tubular polymeric catheter.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
FIG. 1 is a simplified flow chart depicting stages in a method according to an embodiment of the present invention;
FIG. 2 is a simplified cross-sectional side view depiction of a thin metallic film angioplasty balloon and associated polymeric tubular catheter relevant to embodiments of the present invention;
FIGs. 3A and 3B are simplified cross-sectional depictions of a portion of a thin metallic film angioplasty balloon and a portion of an associated polymeric tubular catheter prior to the application of heat and/or pressure during a method according to an embodiment of the present invention;
FIGs. 4A and 4B are a simplified depictions of a portion of a thin metallic film angioplasty balloon and a portion of an associated polymeric tubular catheter following the application of heat and/or pressure during a method according to an embodiment of the present invention; and
FIG. 5 is a medical device (namely, an angioplasty balloon and attached polymeric catheter) according to an embodiment of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict selected exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

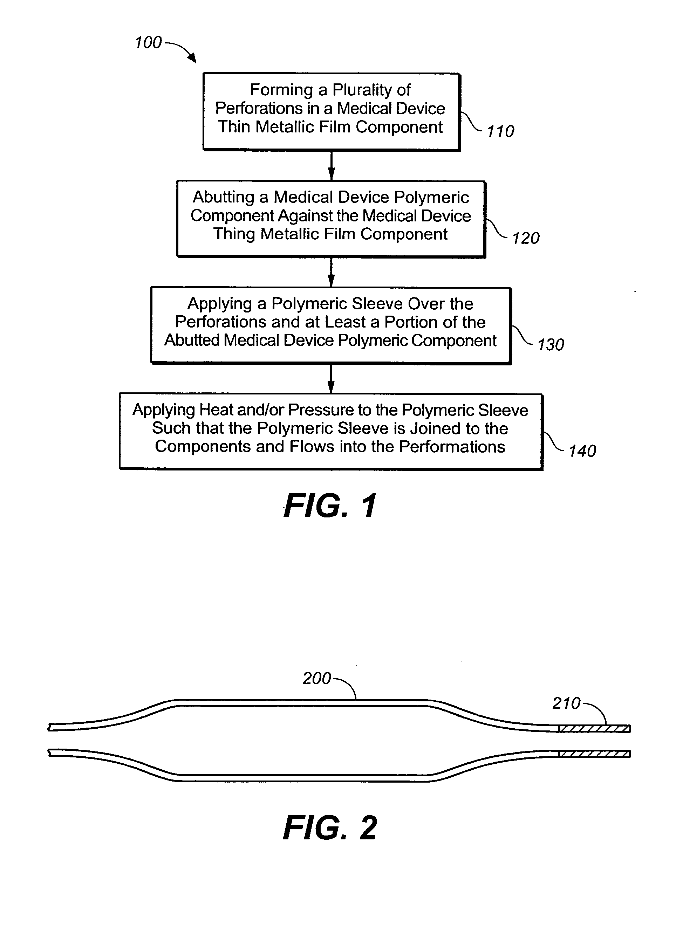

FIG. 1 is a flow diagram depicting stages in method 100 for attaching a medical device thin metallic film component to a medical device polymeric component according to an embodiment of the present invention. The medical device thin metallic film component and medical device polymeric component can be any suitable medical device components known to one skilled in the art including, for example, stent components and angioplasty components.

For illustrative purposes only, method 100 will be described with respect to a medical device thin metallic film angioplasty balloon and a medical device tubular polymeric catheter associated with the balloon. FIG. 2 is a simplified cross-sectional side view depiction of a thin metallic film angioplasty balloon 200 and associated polymeric tubular catheter 210 relevant to embodiments of the present invention.

Referring to FIG. 1, method 100 includes, at step 110, forming a plurality of perforations in the medical device thin metallic film component. As previously mentioned, the medical device thin metallic film component can be any suitable medical device thin metallic component including, for example, those formed of aluminum, chromium, cobalt, nickel, niobium, silver, gold, magnesium, manganese, molybdenum, palladium, platinum, scandium, tantalum, titanium, vanadium, zirconium, and combinations thereof such as stainless steel and nitinol. The thickness of the medical device thin metallic film is, for example, in the range of 0.5 microns to 20 microns.

Once apprised of the present disclosure, a suitable number and size for the perforations can be readily determined by one skilled in the art. A typical non-limiting range for the diameter of circular perorations is 5 microns to 50 microns. Moreover, the perforations can be any suitable shape including, for example, circular, rectangular, oval, and serpentine. A non-limiting example of the number of perforation is a number in the range of 1 perforation to 40 perforations.

The perforations can be formed using any suitable conventional technique including, for example, laser-based and etching perforation techniques.

Next, at step 120, the medical device polymeric component is operatively abutted against the medical device thin film metallic component. The medical device polymeric component can be relatively thin, (having, for example, a thickness in the range of 2.5 microns to 3.0 microns). Such relatively thin thicknesses of medical device polymeric components are not readily adhered to medical device thin metallic film components using conventional adhesives but can be securely adhered using methods according to the present invention. Moreover, methods according to the present invention provide a medical device with a relatively low profile despite the presence of an attachment region medical device polymeric component.

Subsequently a polymeric sleeve is applied over the perforations and over at least a portion of the abutted medical device polymeric component, as set forth in step 130 of FIG. 1. A typical range for the thickness of the polymeric sleeve is in the range of 0.0001 inches to 0.003 inches. Suitable polymers for use in the polymeric sleeve include, for example, nylon and Pebax. The polymeric sleeve can overlap the medical device thin metallic film and the medical device polymeric component by any suitable amount including, for example, a length in the range of 1mm to 10mm. For medical devices that require flexibility, such as those that include an angioplasty balloon and tubular polymeric catheter, the polymeric sleeve can beneficially be formed of a flexible polymer with a Durameter value in the range of 10 Durameter to 40 Durameter. Once apprised of the present disclosure, one skilled in the art can readily select flexible polymeric materials suitable for use in embodiments of the present invention.

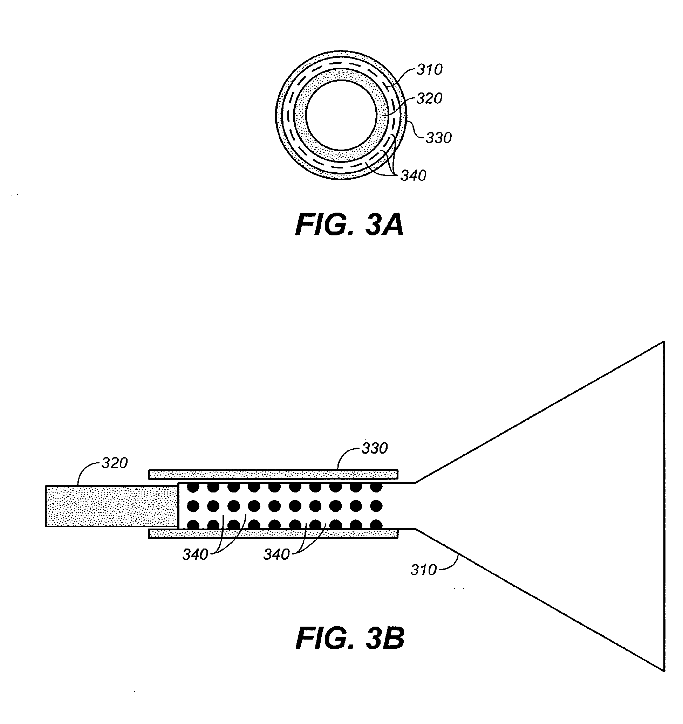

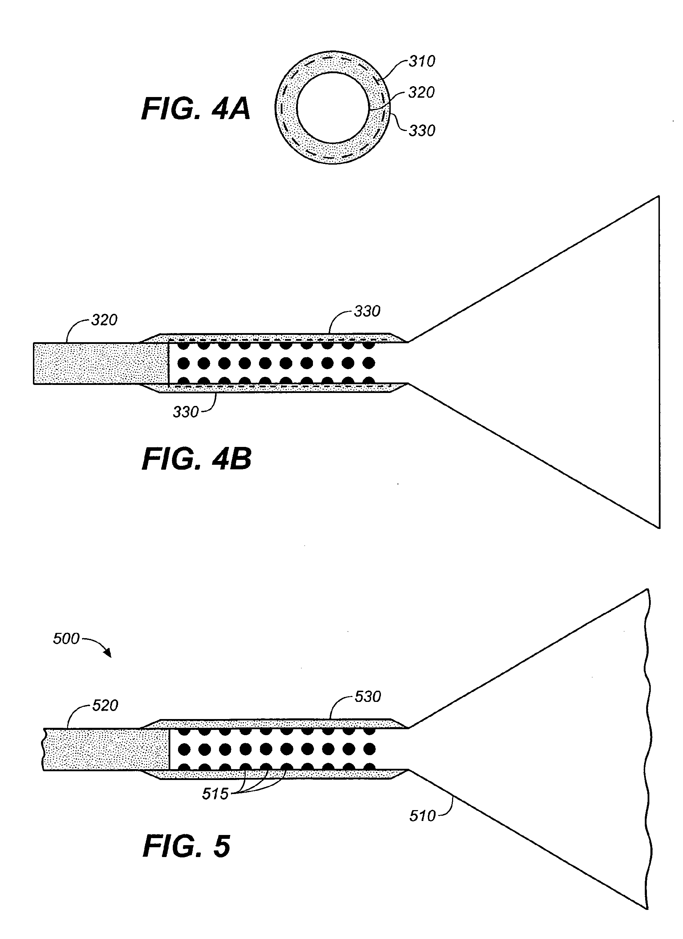

The polymeric sleeve can be applied using any suitable technique including mechanical application of a preformed sleeve or the deposition of a polymeric material to form a sleeve on the surface of the medical device thin metallic film component and the medical device polymeric component. Moreover the term "sleeve" refers generally to a covering or layer and also to a sheath (as illustrated in FIGs. 3A, 3B, 4A and 4B).

FIGs. 3A and 3B are simplified depictions of a portion of a thin metallic film angioplasty balloon 310, a portion of an associated polymeric tubular catheter 320, and a polymeric sleeve 330 prior to the application of heat and/or pressure during a method according to an embodiment of the present invention. FIG. 3 also depicts a plurality of perforations 340 that were previously formed in thin metallic film angioplasty balloon. FIGs. 3A and 3B, therefore, serve to generally illustrate method 100 following step 130.

Referring again to FIG. 1, at least one of heat and pressure is subsequently applied to the polymer sleeve such that the polymer sleeve is joined to the medical device metallic film and the portion of the abutted medical device polymeric component and such that the polymer sleeve flows into the perforations. This joining results in the medical device thin metallic film component being securely attached to the medical device polymeric component. See step 140 of FIG. 1. If desired, the polymeric tubular catheter can have been positioned such that it extended into the thin metallic film angioplasty balloon and underlined the plurality of perforations. In such a circumstance, the polymeric sleeve can also join with the portion(s) of the polymeric tubular catheter that underlies the perforations during the application of heat and/or pressure.

Any suitable temperature and pressure can be employed in step 140. For example, a temperature equivalent to the glass transition temperature of the polymeric sleeve and medical device polymeric component can be employed. Application of suitable pressure will result in the polymeric sleeve flowing into (intruding into) the perforations and becoming joined with the medical device polymeric component. In this respect, it is beneficial to employ a polymeric sleeve that is made of the same polymeric material as the medical device polymeric component. The temperature applied in step 240 can, for example, be in the range of 150F to 250F.

FIGs. 4A and 4B are simplified depictions of a portion of a thin metallic film angioplasty balloon 310, a portion of an associated polymeric tubular catheter 320 and a polymeric sleeve 330 following the application of heat and/or pressure during a method according to an embodiment of the present invention. FIGs. 4A and 4B, therefore, serve to generally illustrate method 100 following step 140.

FIG. 5 is simplified cross-sectional view of a medical device 500 (depicted as an angioplasty balloon and attached polymeric catheter for illustrative purposes only) according to an embodiment of the present invention. Medical device 500 includes a medical device thin metallic film component 510 (such as a thin metallic angioplasty balloon), with perforations 515 therethrough, and a medical device polymeric component 520 (e.g., a polymeric tubular catheter) abutting the thin metallic film component. The medical device also includes a polymeric sleeve 530 covering the perforations and at least a portion of the polymeric component abutting the thin metallic film component. Moreover, the polymeric sleeve intrudes into the perforations and joins the thin metallic film component to the polymeric component.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and methods within the scope of these claims and their equivalents be covered thereby.

## Claims

1. A medical device comprising:
a medical device metallic substrate component with perforations therethrough;
a medical device polymeric component abutting metallic substrate component; and
a polymeric sleeve covering the perforations and at least a portion of the polymeric component abutting the metallic substrate component, the polymeric sleeve intruding into the perforations and joining the medical device metallic substrate component to the medical device polymeric component.

2. The medical device of claim 1 wherein the medical device polymeric component is a tubular polymeric catheter.

3. The medical device of claim 1 wherein the medical device polymeric component and the polymeric sleeve are formed of the same polymeric material.

4. A method for attaching a medical device metallic substrate component to a medical device polymeric component comprising:
forming a plurality of perforations in the medical device metallic substrate component;
abutting the medical device polymeric component against the medical device metallic substrate component;
applying a polymeric sleeve over the perforations and at least a portion of the abutted medical device polymeric component;
applying at least one of heat and pressure to the polymeric sleeve such that the polymer sleeve is joined to the medical device metallic substrate and the portion of the abutted medical device polymeric component and such that the polymeric sleeve flows into the perforations, thereby attaching the medical device metallic substrate component to the medical device polymeric component.

5. The method of claim 4 wherein the medical device polymeric component is a tubular polymeric catheter.

6. The method of claim 4 wherein the medical device polymeric component and the polymeric sleeve are formed of the same polymeric material.
